(19) **Europäisches Patentamt**
**European Patent Office**
**Office européen des brevets**

(11) **EP 3 218 055 B2**

(12) # NEUE EUROPÄISCHE PATENTSCHRIFT
## Nach dem Einspruchsverfahren

(45) Veröffentlichungstag und Bekanntmachung des Hinweises auf die Entscheidung über den Einspruch:
**28.06.2023 Patentblatt 2023/26**

(45) Hinweis auf die Patenterteilung:
**15.07.2020 Patentblatt 2020/29**

(21) Anmeldenummer: **14799732.4**

(22) Anmeldetag: **13.11.2014**

(51) Internationale Patentklassifikation (IPC):
**A61N 2/02** (2006.01)  **A61N 2/00** (2006.01)

(52) Gemeinsame Patentklassifikation (CPC):
**A61N 2/02; A61N 2/004**

(86) Internationale Anmeldenummer:
**PCT/EP2014/074497**

(87) Internationale Veröffentlichungsnummer:
**WO 2016/074726 (19.05.2016 Gazette 2016/20)**

(54) **MAGNETFELDTHERAPIEVORRICHTUNG SOWIE VERFAHREN ZUR ERZEUGUNG EINES MAGNETISCHEN FELDES**

MAGNETIC FIELD THERAPY APPARATUS AND METHOD OF GENERATING A MAGNETIC FIELD

APPAREIL DE THÉRAPIE PAR CHAMP MAGNÉTIQUE ET MÉTHODE DE GÉNÉRATION D'UN CHAMP MAGNÉTIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**20.09.2017 Patentblatt 2017/38**

(73) Patentinhaber: **Pfeiffer, Knut**
**80337 München (DE)**

(72) Erfinder: **Pfeiffer, Knut**
**80337 München (DE)**

(74) Vertreter: **Meissner Bolte Partnerschaft mbB**
**Patentanwälte Rechtsanwälte**
**Postfach 86 06 24**
**81633 München (DE)**

(56) Entgegenhaltungen:
**EP-A2- 1 138 348   DE-A1-102009 017 229**

**Beschreibung**

Die Erfindung betrifft eine Magnetfeldtherapievorrichtung, umfassend eine Magnetfelderzeugungsvorrichtung zum Erzeugen eines auf einem Patienten applizierbaren magnetischen Feldes und eine Steuervorrichtung zum Steuern einer Feldstärke des magnetischen Feldes derart, dass eine schrittweise Erhöhung der magnetischen Feldstärke stattfindet, sowie ein Verfahren zur Erzeugung eines magnetischen Feldes, bei dem eine magnetische Feldstärke schrittweise erhöht wird.

Eine gattungsgemäße Magnetfeldtherapievorrichtung ist aus DE 10 2009 017 229 A1 bekannt.

Die Vorrichtung gemäß DE 10 2009 017 229 A1 umfasst Mittel, die derart ausgebildet sind, dass ein magnetisches Signal generierbar und bei einem Patienten applizierbar ist. Dabei umfasst der zeitliche Verlauf des generierbaren magnetischen Signals eine schrittweise Erhöhung der magnetischen Feldstärke. Die magnetische Feldstärke erhöht sich nach bestimmten Zeitintervallen um bestimmte Feldstärke-Inkremente. Insgesamt hat es sich gezeigt, dass durch die Vorrichtung gemäß DE 10 2009 017 229 A1 das Wohlbefinden von Patienten gesteigert werden kann. Die Wirksamkeit der Magnetfeldtherapievorrichtung wird jedoch noch als verbesserungswürdig angesehen.

Es ist daher Aufgabe der Erfindung, die Wirksamkeit einer Magnetfeldtherapievorrichtung weiter zu steigern, insbesondere das Wohlbefinden von Patienten zu steigern und/oder Heilungsverläufe zu beschleunigen bzw. Krankheiten entgegenzuwirken. Diese Aufgabe wird durch eine Magnetfeldtherapievorrichtung nach Anspruch 1 gelöst.

Insbesondere wird die Aufgabe durch eine Magnetfeldtherapievorrichtung gelöst, umfassend eine Magnetfelderzeugungsvorrichtung zum Erzeugen eines auf einen Patienten applizierbaren magnetischen Feldes und eine Steuervorrichtung zum Steuern einer Feldstärke des magnetischen Feldes derart, dass während eines ersten Zeitraums eine schrittweise Erhöhung der magnetischen Feldstärke stattfindet und während eines (sich an den ersten Zeitraum anschließenden) zweiten Zeitraums eine konstante Feldstärke eingestellt ist, wobei das Verhältnis des ersten Zeitraums zum zweiten Zeitraum dem Goldenen Schritt +/- 0,1, vorzugsweise +/- 0,01, noch weiter vorzugsweise +/- 0,005, noch weiter vorzugsweise +/-0,001, noch weiter vorzugsweise (exakt) dem Goldenen Schnitt entspricht. Weitere Merkmale ergeben sich aus Anspruch 1.

Ein Kerngedanke der Erfindung liegt darin, ein magnetisches Signal mit einer konstanten Feldstärke nach einem Signal mit einer schrittweise erhöhten magnetischen Feldstärke bereitzustellen. Ein Verhältnis des Zeitraums, in dem eine schrittweise Erhöhung der magnetischen Feldstärke stattfindet, und des Zeitraums, in dem eine konstante Feldstärke eingestellt ist, wird gemäß dem Goldenen Schnitt eingestellt. Bei einer derartigen Einstellung der Zeiträume hat es sich auf überraschende Art und Weise gezeigt, dass das Wohlbefinden des Patienten besonders effektiv gesteigert werden kann. Weiterhin konnten Heilungsprozesse beschleunigt werden sowie Erkrankungen entgegengewirkt werden. Insbesondere hat es sich gezeigt, dass effektiv Stress und stressinduzierten Erkrankungen entgegengewirkt werden kann. Muskelverspannungen konnten gelindert werden. Aufmerksamkeitsdefizite bzw. Lernschwächen konnten reduziert werden. Weiterhin hat sich die Magnetfeldtherapievorrichtung auch für die Bekämpfung von Rheuma und Migräne als effektiv erwiesen.

Die Verwendung der Magnetfeldtherapievorrichtung für eine oder mehrere der obigen Indikationen wird als eigenständiger Erfindungsgedanke hiermit offenbart und beansprucht.

Als Goldener Schnitt wird üblicherweise (wie auch im vorliegenden Zusammenhang) das Teilungsverhältnis einer Größe (im vorliegenden Fall einer Zeit), bei der das Verhältnis des Ganzen zu seinem größeren Teil (im vorliegenden Fall dem ersten Zeitraum) dem Verhältnis des größeren zum kleineren Teil (im vorliegenden Fall dem zweiten Zeitraum) entspricht. Als Formel ausgedrückt (mit $T_1$ als ersten Zeitraum und $T_2$ als zweiten Zeitraum) gilt:

$$(T_1 + T_2) / T_1 = T_1 / T_2$$

Der Goldene Schnitt ist eine irrationale Zahl, d.h. eine Zahl, die sich nicht als Bruch ganzer Zahlen darstellen lässt. Als mathematisches Symbol für diese Zahl wird (wie auch im vorliegenden Zusammenhang) der griechische Buchstabe $\Phi$ verwendet. Als irrationale Zahl lässt sich $\Phi$ nicht als endlicher Dezimalbruch darstellen. Auf die zehnte Nachkommastelle gerundet ergibt sich ein Wert von $\Phi = 1,6180339887$.

Die vorliegende Erfindung basiert grundsätzlich auf der Erkenntnis, dass zahlreiche (anatomische) Strukturen des menschlichen Körpers im goldenen Schnitt angelegt sind. Daraus lässt sich schließen, dass sich räumliche Proportionen gemäß dem goldenen Schnitt vorteilhaft auswirken. Die Übertragung dieser Erkenntnis auf eine zeitliche Proportion, d.h. die erfindungsgemäße Einstellung des Verhältnisses der zwei Zeiträume nach dem goldenen Schnitt, resultiert letztlich aus intuitiven Überlegungen. Die Wirksamkeit wurde zunächst vermutet, konnte dann jedoch auch aufgrund der Reaktion von Patienten überprüft werden.

Die schrittweise Erhöhung im ersten Zeitraum erfasst vorzugsweise mindestens drei, weiter vorzugsweise mindestens fünf Erhöhungsschritte. Eine Obergrenze der Erhöhungsschritte kann beispielsweise 20 Schritte oder 8 Schritte betragen. Insgesamt kann ein Treppensignal ausgebildet werden, wobei jede Treppenstufe einem Erhöhungsschritt entspricht. Der Zeitraum zwischen zwei aufeinanderfolgenden Erhöhungsschritten kann konstant sein (oder variieren). Die Erhöhung der Feldstärke kann ebenfalls konstant sein (oder variieren).

In einer konkreten Ausführungsform kann mindestens eine Batterie (insbesondere mindestens ein Acku) vorgesehen sein, um einen Gleichstrom zur Erzeugung des Magnetfeldes bereitzustellen. Dadurch kann die Magnetfeldstärke besonders exakt eingestellt werden und insbesondere kurze Stromspitzen, die bei einer Gleichrichtung eines Netz-Wechselstroms auftreten, können vermieden werden. Insgesamt wird die Wirksamkeit der Magnetfeldtherapievorrichtung dadurch erhöht.

In einer Weiterbildung ist die Steuervorrichtung zum Nachregeln der Magnetfeldstärke ausgebildet. Auch durch eine derartige Maßnahme wird die Genauigkeit und Konstanz des applizierten Magnetfeldes verbessert, was die Wirksamkeit der Magnetfeldtherapievorrichtung erhöht. Ein Magnetometer kann vorgesehen sein, um das applizierte Magnetfeld zu messen und der Regelung zu Grunde zu legen. Beispielsweise kann es sich bei dem Magnetometer um einen Hall-Sensor handeln.

In einer konkreten Ausführungsform ist eine Abschirmungseinrichtung zum Abschirmen von elektromagnetischen Feldern vorgesehen. Vorzugsweise umfasst die Abschirmungseinrichtung mindestens ein Lochblech. Alternativ oder zusätzlich kann die Abschirmungseinrichtung eine Stoffbahn umfassen. In einer konkreten Weiterbildung kann die Abschirmungseinrichtung (zumindest teilweise) als Zelt ausgebildet sein. Weiterhin kann die Abschirmung als Einhausung ausgestaltet sein, wobei sich der Behandlungsplatz für den Patienten innerhalb der Einhausung befindet. Insgesamt können durch die Abschirmung äußere elektromagnetische Felder vom Patienten bzw. vom Behandlungsplatz, an dem das generierte magnetische Signal appliziert wird, effektiv abgeschirmt werden. Mit der Abschirmung können äußere elektromagnetische Felder wie beispielsweise Elektrosmog blockiert werden, die die Behandlung bzw. die Applikation des generierten elektromagnetischen Signals stören könnten. Auch durch eine derartige Maßnahme wird die Wirksamkeit der Magnetfeldtherapievorrichtung weiter gesteigert.

Besonders bevorzugt ist die Abschirmungseinrichtung aus Lochblechen aufgebaut (oder umfasst derartige Lochbleche). Dadurch wird auf synergistische Weise einerseits eine elektromagnetische Abschirmung erreicht und andererseits eine Schallreduzierung, da die Lochbleche als Helmholtz-Resonatoren wirken. Dazu können die einzelnen Löcher einen kreisförmigen (oder alternativ elliptischen oder rechteckförmigen) Querschnitt aufweisen. Insgesamt ist eine Vielzahl von Löchern vorgesehen, vorzugsweise mindestens 100, weiter vorzugsweise mindestens 1.000. Eine durch die Löcher definierte Fläche der Lochbleche kann 5% bis 50%, vorzugsweise 7% bis 20% der Gesamtfläche der Lochbleche betragen. Die Löcher können einen Durchmesser von 2 mm bis 5 mm aufweisen. Die Weiterbildung der Magnetfeldtherapievorrichtung umfassend mindestens ein Lochblech bzw. die Ausbildung einer Abschirmung aus mindestens einem Lochblech wird als eigenständiger, unabhängiger Erfindungsgedanke beansprucht (insbesondere auch unabhängig von der Realisierung eines Goldenen Schnittes betreffend den ersten bzw. zweiten Zeitraum). Insbesondere wird eine Kombination aus einem magnetischen Signal, das schrittweise erhöht wird (mit den vorangehenden und folgenden Weiterbildungen) zusammen mit einer Abschirmung, die ein Lochblech umfasst oder sich (zumindest teilweise) aus Lochblechen zusammensetzt, als eigenständiger Erfindungsgedanke hiermit beansprucht und offenbart.

Der zeitliche Verlauf der magnetischen Feldstärke im ersten Zeitraum kann eine Erhöhung der magnetischen Feldstärke um bestimmte (insbesondere konstante) Feldstärke-Inkremente nach bestimmten (insbesondere konstanten) Zeitintervallen umfassen. In einer alternativen Ausgestaltung ist es möglich, dass die magnetische Feldstärke nach gleichen Zeitintervallen um verschiedene Feldstärke-Inkremente erhöht wird.

Die generierbaren magnetischen Feldstärken können zwischen 0 $\mu$T und 150 $\mu$T liegen. Alternativ oder zusätzlich können die Erhöhungs-Inkremente für die magnetische Feldstärke zwischen 2 $\mu$T und 60 $\mu$T liegen. Alternativ oder zusätzlich können die Zeitabstände zwischen den Erhöhungschritten zwischen 5 und 8 Sekunden liegen. Es hat sich gezeigt, dass mit derartigen Zeitverläufen des Magnetfeldes eine Steigerung des Wohlbefindens des Patienten besonders effektiv erreicht werden kann.

Im Allgemeinen liegt die generierbare magnetische Feldstärke vorzugsweise in der Größenordnung des Erdmagnetfeldes. Die applizierbare magnetische Feldstärke weist einen im Allgemeinen stufen- bzw. treppenartigen Verlauf auf.

Die Feldstärke im zweiten Zeitraum beträgt vorzugsweise 15 bis 40 $\mu$T, 20 bis 30 $\mu$T, weiter vorzugsweise 25,12 $\mu$T. Bei einer derartigen Feldstärke wird ebenfalls das Wohlbefinden des Patienten effektiv gesteigert.

Der erste Zeitraum kann 28 bis 58 Sekunden, vorzugsweise 38 bis 48 Sekunden, insbesondere 43 Sekunden lang sein. Ein Zyklus aus erstem und zweitem Zeitraum kann wiederholt werden. Vorzugsweise erfolgt eine Wiederholung des Zyklus (umfassend den ersten und den zweiten Zeitraum) in einer ersten Ausführungsform 40 bis 58mal, insbesondere 49mal. In einer zweiten Ausführungsform erfolgen 30 bis 42, vorzugsweise 37 Wiederholungen.

Eine Gesamtdauer der Applikation kann in einer ersten Ausführungsform 50 bis 66 Minuten, insbesondere 58,09 Minuten dauern. In einer zweiten Ausführungsform kann die Applikationsdauer 30 bis 42 Minuten, insbesondere 36,75 Minuten dauern. Durch derartige Werte wird das Wohlbefinden des Patienten effektiv gesteigert.

Die Feldstärke im zweiten Zeitraum ist 5 bis 13mal, insbesondere 8 bis 10mal so groß wie die Feldstärke einer minimalen Stufe im ersten Zeitraum. Alternativ oder zusätzlich ist die Feldstärke im zweiten Zeitraum 0,15 bis 0,40, insbesondere 0,25 bis 0,3mal so groß wie die Feldstärke einer maximalen Stufe im ersten Zeitraum.

In einer konkreten Ausführungsform umfasst die Magnetfelderzeugungsvorrichtung mindestens eine, vorzugsweise mindestens zwei oder genau zwei Leiterspule(n). Weiter vorzugsweise ist eine erste Spule elliptisch oder oval ausgebildet. Alternativ oder zusätzlich kann eine erste Spule einen (längeren) Durchmesser von 1,5 bis 2,0 Meter, insbesondere 1,70 bis 1,80 Meter aufweisen. Alternativ oder zusätzlich kann eine zweite Spule vorgesehen sein, die vorzugsweise einen (etwa) runden Querschnitt aufweist. Alternativ oder zusätzlich kann die zweite Spule einen Querschnitt aufweisen, der kleiner ist als der kleinere Querschnitt der ersten Spule. Ein zentraler Bereich einer oder mehrerer der Spulen kann im Bauchbereich (solar plexus) des Patienten angeordnet sein. Bei einer runden Ausgestaltung der Spule ist der zentrale Bereich durch den Mittelpunkt definiert. Bei einer elliptischen Ausgestaltung der Spule ist der zentrale Bereich durch einen Mittelpunkt einer Verbindungslinie der beiden Ellipsen-Brennpunkte definiert. Ein längerer Durchmesser der ersten Spule kann (in etwa) der Größe eines Patienten entsprechen. Die zweite Spule ist vorzugsweise (vollständig) innerhalb eines zentralen Drittels einer Liegefläche des Patienten angeordnet.

Vorgeschlagen wird weiterhin ein nicht-beanspruchtes Verfahren zur Erzeugung eines Magnetfeldes (insbesondere unter Verwendung der Magnetfeldtherapievorrichtung der oben beschriebenen Art), bei dem in einem ersten Zeitraum eine magnetische Feldstärke schrittweise erhöht wird und in einem zweiten Zeitraum eine konstante magnetische Feldstärke erzeugt wird, wobei ein Verhältnis von erstem zu zweitem Zeitraum dem Goldenen Schnitt +/-0,1, vorzugsweise +/- 0,01, noch weiter vorzugsweise +/- 0,005, noch weiter vorzugsweise +/- 0,001 entspricht. Die Feldstärke im zweiten Zeitraum ist 5 bis 13mal, insbesondere 8 bis 10mal so groß wie die Feldstärke einer minimalen Stufe im ersten Zeitraum. Alternativ oder zusätzlich ist die Feldstärke im zweiten Zeitraum 0,15 bis 0,40, insbesondere 0,25 bis 0,3mal so groß wie die Feldstärke einer maximalen Stufe im ersten Zeitraum. Durch die Applikation eines derartigen Verfahrens auf einen Patienten kann dessen Wohlbefinden effektiv gesteigert werden. Insbesondere hat es sich gezeigt, dass Stress und stressinduzierte Erkrankungen, Muskelverspannungen, Aufmerksamkeitsdefizite bzw. Lernschwächen, Rheuma und Migräne wirksam behandelt werden können.

Vorzugsweise wird die magnetische Feldstärke im ersten Zeitraum nach bestimmten (insbesondere konstanten) Zeitintervallen um bestimmte Feldstärke-Inkremente erhöht. Weiter vorzugsweise erfolgt die Erhöhung der magnetischen Feldstärke im ersten Zeitraum jeweils nach gleichen Zeitintervallen und/oder wird um gleiche Feldstärke-Inkremente erhöht. In einer konkreten Ausführungsform wird die magnetische Feldstärke nach den gleichen Zeitintervallen um verschiedene Feldstärke-Inkremente erhöht.

Vorzugsweise wird die Feldstärke nachgeregelt. Dazu kann eine Messung der Magnetfeldstärke, beispielsweise durch einen Hall-Sensor erfolgen.

Die Feldstärke im zweiten Zeitraum kann auf einen Wert zwischen 15 bis 40 $\mu$T, vorzugsweise 20 bis 30 $\mu$T, weiter vorzugsweise 25,12 $\mu$T betragen. Der erste Zeitraum kann auf 28 bis 58 Sekunden, vorzugsweise 38 bis 48 Sekunden, insbesondere 43,96 Sekunden eingestellt werden. Ein Zyklus umfassend den ersten und den zweiten Zeitraum kann wiederholt werden. Ein Zyklus kann vorzugsweise 40 bis 58mal, insbesondere 49mal wiederholt werden. In einer alternativen Ausführungsform kann der Zyklus 30 bis 42mal, vorzugsweise 31mal wiederholt werden. Die Gesamtdauer der Applikation kann 50 bis 66 Minuten, insbesondere 58,09 Minuten andauern. In einer alternativen Ausführungsform kann die Gesamtdauer der Applikation 30 bis 42 Minuten, vorzugsweise 36,75 Minuten andauern.

Der Strom zur Erzeugung des Magnetfeldes wird vorzugsweise aus einer Batterie gespeist.

Weitere Ausführungsformen ergeben sich aus den Unteransprüchen.

Nachfolgend wird die Erfindung auch hinsichtlich weiterer Merkmale und Vorteile anhand eines Ausführungsbeispiels beschrieben, das anhand der Abbildungen näher erläutert wird.

Hierbei zeigen:

Fig. 1 einen erfindungsgemäßen Verlauf der magnetischen Feldstärke B (t),

Fig. 2 einen erfindungsgemäßen Applikator zur Applikation des Magnetfeldes, und

Fig. 3 eine erfindungsgemäße Magnetfeldtherapievorrichtung.

In der nachfolgenden Beschreibung werden für gleiche und gleichwirkende Teile dieselben Bezugsziffern verwendet.

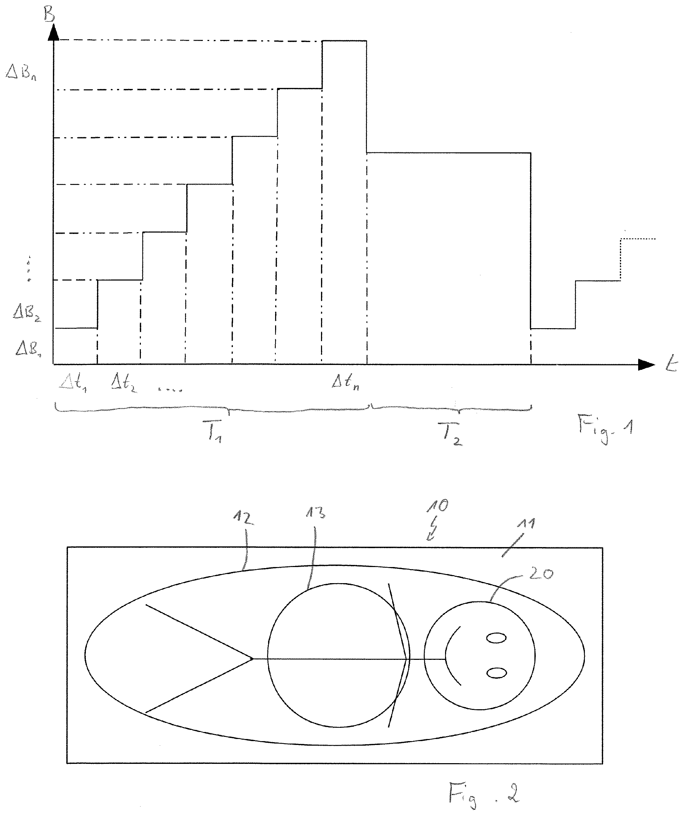

Fig. 1 zeigt ein mit einer erfindungsgemäßen Vorrichtung erzeugtes magnetisches Signal B (t). Die magnetische Feldstärke B (t) erhöht sich im Zeitverlauf stufen- bzw. treppenartig. Nach Zeitintervallen $\Delta t_1$, $\Delta t_2$ ... $\Delta t_n$ erhöht sich die Feldstärke um Feldstärke-Inkremente $\Delta B_1$, $\Delta B_2$ ... $\Delta B_n$. Eine nicht gezeigte Steuervorrichtung steuert die Erzeugung der magnetischen Feldstärke und deren zeitlichen Verlauf B (t). Ein wesentlicher Bestandteil der Steuereinheit ist ein programmierbares Modul, mit dem ein treppenförmiges Feldstärkesignal B (t) innerhalb eines ersten Zeitraums $T_1$ sowie ein konstantes Feldstärkesignal innerhalb eines Zeitraums $T_2$ erzeugt werden kann. Beispielsweise wird folgender Signalverlauf erzeugt:

| | |
|---|---|
| 6,28 Sekunden | B=3,14 μT, dann |
| 6,28 Sekunden | B=9,42 μT, dann |
| 6,28 Sekunden | B=15,7 μT, dann |
| 6,28 Sekunden | B=18,84 μT, dann |
| 6,28 Sekunden | B=25,12 μT, dann |
| 6,28 Sekunden | B=37,68 μT, dann |
| 6,28 Sekunden | B=94,20 μT, dann |
| 27,17 Sekunden | B=25,12 μT. |

Es gilt $T_1$=43,96 Sekunden sowie $T_2$=27,17 Sekunden. Das Verhältnis $T_1$:$T_2$=1,62 (gerundet). Weiterhin ist die Steuervorrichtung so programmiert, dass 49 Wiederholungen durchgeführt werden. Die Applikationszeit dauert damit insgesamt 58,09 Minuten. Ein Zyklus aus $T_1$ und $T_2$ dauert 71,13 Sekunden.

Alternativ können auch 31 Wiederholungen des Zyklus $T_1$+$T_2$ stattfinden, was einer gesamten Applikationszeit von 36,75 Minuten entspricht.

Die magnetischen Feldstärken liegen (sowohl im Zeitraum $T_1$ als auch im Zeitraum $T_2$) zwischen 3 und 100 μT, also in der Größenordnung des Erdmagnetfeldes. Nach Erreichen der maximalen Feldstärke von 94,20 μT wird die Feldstärke bei Beendigung des Zeitraums $T_1$ auf 25,12 μT reduziert. Insgesamt hat es sich gezeigt, dass mit diesem Signalverlauf eine besonders gute Steigerung des Wohlbefindens des Patienten erreicht werden kann.

Fig. 2 zeigt einen erfindungsgemäßen Applikator 10, auf dem ein Patient 20 positioniert ist. Der Applikator 10 umfasst eine Matte 11, in die eine erste Leiterspule 12 (mit ovalem bzw. elliptischem Querschnitt) und eine zweite Leiterspule 13 (mit rundem Querschnitt) eingelassen ist. Die Matte 10 hat eine Oberfläche von (ca.) 220 cm * 120 cm und ist (ungefähr) 2 bis 3 cm dick und umfasst einen Schaumstoffkörper. In diesen Schaumstoff können die Leiterspulen 12, 13 eingearbeitet sein. Die erste Spule 12 mit dem ovalen Querschnitt hat eine größere Querschnittsfläche. Die Querschnittsfläche ist derart gestaltet, dass der gesamte Körperbereich des Patienten abgedeckt werden kann, so dass im Bereich des gesamten Körpers des Patienten das Magnetfeld appliziert werden kann.

Beispielsweise ist ein längerer Durchmesser der Spule 12 190 bis 220 cm lang. Ein kürzerer Durchmesser ist beispielsweise 80 bis 110 cm lang.

Die kleinere Spule 13 mit dem runden Querschnitt ist derart angeordnet, dass sie etwa den Bauchbzw. Torso-Bereich des Patienten (insbesondere den solar plexus) abdeckt. Dazu ist der Durchmesser der kleinen Spule 13 kleiner (beispielsweise um mindestens 5% oder mindestens 10%) als der kürzere Durchmesser der ersten Spule 12. Das Symmetriezentrum beider Spulenquerschnitte liegt (in etwa) im solar-plexus-Bereich. Mit den Spulen 12, 13 kann einerseits der gesamte Körper des Patienten abgedeckt werden, andererseits kann im solar-plexus-Bereich bzw. Torso-Bereich ein überlagertes Magnetfeld generiert werden.

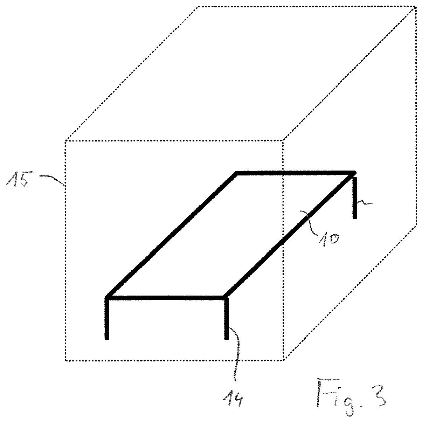

In Fig. 3 ist der Applikator 10 auf eine Patientenliege 14 platziert. Die Patientenliege 14 ist von einer Abschirmung 15 umgeben, so dass äußere elektromagnetische Felder, beispielsweise Elektrosmog, von therapierten Patienten abgeschirmt werden können. Mit der Abschirmung 15 kann vermieden werden, dass die Therapie des Patienten durch äußere elektromagnetische Felder beeinflusst bzw. gestört wird.

Es kann also erreicht werden, dass sich der gesamte Körper eines Patienten im Einflussbereich des generierten Magnetfeldes befindet, ohne äußere elektromagnetische Störfelder. Die erfindungsgemäße Vorrichtung erlaubt somit eine Ganzkörperbehandlung, wobei die therapeutische Wirkung durch die geeignete Modulation der Stromstärke in den Leiterspulen und damit der magnetischen Feldstärke und auch der induzierten elektrischen Felder erreicht wird. Dabei wird die Stromstärke für die Leiterspulen durch die Steuervorrichtung derart abgestimmt, dass die gewünschten Magnetfeldstärken erzeugt werden können.

Beispielsweise können in der Steuervorrichtung entsprechende Programme hinterlegt (gespeichert) sein, so dass der Verlauf der Magnetfeldstärke, wie beispielsweise in Fig. 1 dargestellt, realisiert werden kann. Es ist möglich, in derselben Vorrichtung zwei oder noch mehr derartige Programme zu hinterlegen (abzuspeichern).

Zur Steuerung der Magnetfeldstärke umfasst die Steuervorrichtung entsprechende (an sich bekannte) Strukturen, wie beispielsweise eine Steuereinheit (z.B. Mikroprozessor) und/oder ein Display und/oder ein Eingabemittel (z.B. Touchscreen) und/oder Signaleinrichtungen wie beispielsweise LEDs oder akustische Signaleinrichtungen und/oder eine Speichereinrichtung (Speicherchip).

Die Abschirmung 16 ist vorzugsweise als Einhausung ausgebildet, besteht also aus mehreren Wänden, die die Liege 14 umgeben. Die Abschirmung 15, insbesondere deren Wände, können aus Lochblechen aufgebaut sein (nicht in den Figuren gezeigt).

Die bereitgestellten Magnetfelder sind vorzugsweise homogen. Insofern Feldstärkewerte angegeben werden, beziehen sich diese vorzugsweise auf einen Wert, der im Zentrum der jeweiligen Spule bzw. einer Auflagefläche für den Patienten gemessen wird.

Die Ausführungsbeispiele, die nicht unter den Schutzumfang der beigefügten Ansprüche fallen, dienen lediglich zu illustrativen Zwecken und sind nicht Gegenstand der gegenwärtigen Erfindung. Die Erfindung wird durch die folgenden Ansprüche definiert.

Bezugszeichenliste

10   Applikator

11 Matte
12 Erste Spule
13 Zweite Spule
14 Patientenliege
15 Abschirmung
20 Patient

**Patentansprüche**

1. Magnetfeldtherapievorrichtung, umfassend eine Magnetfelderzeugungsvorrichtung zum Erzeugen eines auf einen Patienten (20) applizierbaren magnetischen Feldes und eine Steuervorrichtung zum Steuern einer Feldstärke des magnetischen Feldes derart, dass während eines ersten Zeitraums ($T_1$) eine schrittweise Erhöhung der magnetischen Feldstärke stattfindet und während eines zweiten Zeitraums ($T_2$) eine konstante Feldstärke erzeugt wird, wobei das Verhältnis des ersten Zeitraums ($T_1$) zum zweiten Zeitraum ($T_2$) dem Goldenen Schnitt +/- 0,1, vorzugsweise +/- 0,01 entspricht, wobei die Feldstärke im zweiten Zeitraum ($T_2$) 5 bis 13mal, insbesondere 8 bis 10mal so groß ist wie die Feldstärke einer minimalen Stufe im ersten Zeitraum ($T_1$) und/oder 0,15 bis 0,40, insbesondere 0,25 bis 0,30mal so groß ist wie die Feldstärke einer Maximalstufe im ersten Zeitraum ($T_1$).

2. Magnetfeldtherapievorrichtung nach Anspruch 1, **gekennzeichnet durch** mindestens eine Batterie zur Bereitstellung eines Gleichstroms zur Erzeugung des Magnetfeldes.

3. Magnetfeldtherapievorrichtung nach Anspruch 1 oder 2, **dadurch gekennzeichnet, dass** die Steuervorrichtung zum Nachregeln der Magnetfeldstärke ausgebildet ist.

4. Magnetfeldtherapievorrichtung nach einem der vorhergehenden Ansprüche, **gekennzeichnet durch** eine Abschirmungseinrichtung (15) zum Abschirmen von elektromagnetischen Feldern, wobei die Abschirmungseinrichtung (15) vorzugsweise mindestens ein Lochblech und/oder eine Stoffbahn umfasst und/oder zumindest teilweise als Zelt oder Einhausung ausgebildet ist.

5. Magnetfeldtherapievorrichtung nach einem der vorhergehenden Ansprüche, **dadurch gekennzeichnet, dass** im ersten Zeitraum ($T_1$) der zeitliche Verlauf des magnetischen Signals eine Erhöhung der magnetischen Feldstärke um bestimmte Feldstärke-Inkremente ($\Delta B_1, \Delta B_2 ..., \Delta B_n$) nach bestimmten Zeitintervallen ($\Delta t_1, \Delta t_2 ..., \Delta t_n$) umfasst und/oder **dadurch gekennzeichnet, dass** die Erhöhung der magnetischen Feldstärke (B) jeweils nach gleichen Zeitintervallen erfolgt und/oder **dadurch gekennzeichnet, dass** die generierbaren magnetischen Feldstärken zwischen 0 $\mu$T und 150 $\mu$T und/oder die Erhöhungs-Inkremente für die magnetische Feldstärke zwischen 2 $\mu$T und 60 $\mu$T und/oder die Zeitabschnitte zwischen den Erhöhungsschritten zwischen 5 und 8 Sekunden liegen.

6. Magnetfeldtherapievorrichtung nach einem der vorhergehenden Ansprüche, **dadurch gekennzeichnet, dass** die Feldstärke im zweiten Zeitraum ($T_2$) 15 bis 40 $\mu$T, vorzugsweise 20 bis 30 $\mu$T, insbesondere 25,12 $\mu$T beträgt.

7. Magnetfeldtherapievorrichtung nach einem der vorhergehenden Ansprüche, **dadurch gekennzeichnet, dass** der erste Zeitraum ($T_1$) 28 bis 58 Sekunden, vorzugsweise 38 bis 48 Sekunden, insbesondere 43,96 Sekunden lang ist und/oder **dadurch gekennzeichnet, dass** ein Zyklus aus erstem ($T_1$) und zweitem ($T_2$) Zeitraum wiederholt wird, vorzugsweise 40 bis 58mal, insbesondere 49mal oder 30 bis 42mal, insbesondere 37mal und/oder **dadurch gekennzeichnet, dass** die Gesamtdauer der Applikation 50 bis 66 Minuten, insbesondere 58,09 Minuten oder 30 bis 42 Minuten, insbesondere 36,75 Minuten dauert.

8. Magnetfeldtherapievorrichtung nach einem der vorhergehenden Ansprüche, **dadurch gekennzeichnet, dass** die Magnetfelderzeugungsvorrichtung mindestens eine, vorzugsweise mindestens zwei, insbesondere genau zwei, Leiterspule(n) umfasst, wobei eine erste Spule (12) vorzugsweise oval oder elliptisch ist und/oder einen (maximalen) Durchmesser von 1,5 bis 2,0 Meter, vorzugsweise 1,70 bis 1,80 Meter aufweist, und/oder **dadurch gekennzeichnet, dass** eine zweite Spule (13) vorzugsweise einen runden Querschnitt aufweist und/oder vorzugsweise einen Querschnitt aufweist, der kleiner ist als der Querschnitt der ersten Spule (12) und/oder **dadurch gekennzeichnet, dass** ein zentraler Bereich einer oder mehrerer der Spulen (12, 13) im Bauchbereich (solar plexus) des Patienten angeordnet ist.

9. Magnetfeldtherapievorrichtung nach einem der vorhergehenden Ansprüche, **dadurch gekennzeichnet, dass** die Magnetfelderzeugungsvorrichtung eine Matte umfasst, in der vorzugsweise zumindest eine Spule (12, 13) eingelassen ist.

**Claims**

1. A magnetic field therapy apparatus, comprising a magnetic field generating apparatus for generating a magnetic field which can be applied to a patient (20), and a control apparatus for controlling a field strength of the magnetic field such that a stepwise increase of the magnetic field strength takes place during a first time period ($T_1$) and a constant field strength is set during a second period ($T_2$), wherein the ratio of the first period ($T_1$) to the second period (Tz) corresponds to the golden section +/- 0.1, preferably +/- 0.01, wherein the field strength in the second period ($T_2$) is 5 to 13 times, in particular 8 to 10 times, as large as the field strength of a minimal stage in the first time period ($T_1$), and/or 0.15 to 0.40, in particular 0.25 to 0.30, times as large as the field strength of a maximum stage in the first period ($T_1$).

2. A magnetic field therapy apparatus according to claim 1, **characterized by** at least one battery for providing a direct current for generating the magnetic field.

3. A magnetic field therapy apparatus according to claim 1 or 2, **characterized in that** the control apparatus is designed for readjusting the magnetic field strength.

4. A magnetic field therapy apparatus according to one of the preceding claims, **characterized by** a shielding device (15) for shielding electromagnetic fields, wherein the shielding device (15) preferably comprises at least one perforated plate and/or a fabric web and/or is at least partially designed as a tent or housing.

5. A magnetic field therapy apparatus according to one of the preceding claims, **characterized in that**

in the first time period ($T_1$), the time course of the magnetic signal comprises an increase in the magnetic field strength by specific field strength increments ($\Delta B_1$, $\Delta B_2$, ... $\Delta B_n$.) after specific time intervals ($\Delta t_1$, $\Delta t_2$, ... $\Delta t_n$,), and/or **characterized in that** increase in the magnetic field strength (B) respectively takes place after the same time intervals, and/or
**characterized in that**
the generatable magnetic field strengths are between 0 $\mu$T and 150 $\mu$T and/or the incremental increments for the magnetic field strength lie between 2 $\mu$T and 60 $\mu$T and/or the time intervals between the increment steps lie between 5 and 8 seconds.

6. A magnetic field therapy apparatus according to one of the preceding claims, **characterized in that** the field strength in the second period ($T_2$) is 15 to 40 $\mu$T, preferably 20 to 30 $\mu$T, in particular 25.12 $\mu$T.

7. A magnetic field therapy apparatus according to one of the preceding claims, **characterized in that**

the first period ($T_1$) is 28 to 58 seconds, preferably 38 to 48 seconds, in particular 43.96 seconds, and/or
**characterized in that**
a cycle of the first ($T_1$) and the second ($T_2$) period is repeated, preferably 40 to 58 times, in particular 49 times, or 30 to 42 times, in particular 37 times, and/or
**characterized in that**
the total duration of the application lasts 50 to 66 minutes, in particular 58.09 minutes, or 30 to 42 minutes, in particular 36.75 minutes.

8. A magnetic field therapy apparatus according to one of the preceding claims, **characterized in that**

the magnetic field generating apparatus comprises at least one,
preferably at least two, in particular exactly two, conductor coils, wherein a first coil (12) is preferably oval or elliptical and/or has a (maximum) diameter of 1.5 to 2.0 meters, preferably 1.70 to 1.80 meters, and/or
**characterized in that**
the second coil (13) preferably has a round cross-section and/or preferably a cross-section which is smaller than the cross-section of the first coil (12), and/or
**characterized in that**
a central region of one or more of the coils (12, 13) is arranged in the abdominal area (solar plexus) of the patient.

9. A magnetic field therapy apparatus according to one of the preceding claims, **characterized in that** the magnetic field generating apparatus comprises a mat in which preferably at least one coil (12, 13) is embedded.

**Revendications**

1. Dispositif de magnétothérapie, comprenant un dispositif de génération de champ magnétique pour générer un champ magnétique pouvant être appliqué à un patient (20) et un dispositif de commande pour commander une intensité de champ du champ magnétique de telle sorte qu'une augmentation progressive de l'intensité de champ magnétique ait lieu pendant une première période ($T_1$) et qu'une intensité de champ constante soit générée pendant une deuxième période ($T_2$), le rapport entre la première période ($T_1$) et la deuxième période ($T_2$) correspondant au nombre d'or +/- 0,1, de préférence +/- 0,01, l'intensité de champ dans la deuxième période ($T_2$) étant 5 à 13 fois, en particulier 8 à 10 fois plus grande que l'intensité de champ d'un niveau minimal dans la première période ($T_1$) et/ou 0,15 à 0,40, en particulier 0,25 à 0,30 fois plus grande que l'intensité de champ d'un niveau maximal dans la première période ($T_1$).

2. Dispositif de magnétothérapie selon la revendication 1,
   **caractérisé par**
   au moins une batterie pour fournir un courant continu afin de générer le champ magnétique.

3. Dispositif de magnétothérapie selon la revendication 1 ou 2,
   **caractérisé en ce que**
   le dispositif de commande est conçu pour réajuster l'intensité de champ magnétique.

4. Dispositif de magnétothérapie selon l'une des revendications précédentes,
   **caractérisé par**
   un moyen de blindage (15) pour faire écran aux champs électromagnétiques, le moyen de blindage (15) comprenant de préférence au moins une tôle perforée et/ou un pan de tissu et/ou étant conçu au moins partiellement comme une tente ou une enceinte.

5. Dispositif de magnétothérapie selon l'une des revendications précédentes,
   **caractérisé en ce que**,

   dans la première période ($T_1$), l'évolution temporelle du signal magnétique comprend une augmentation de l'intensité de champ magnétique d'incréments d'intensité de champ définis ($\Delta B_1$, $\Delta B_2$ ..., $\Delta B_n$) après des intervalles de temps définis ($\Delta t_1$, $\Delta t_2$ ... , $\Delta t_n$) et/ou
   **caractérisé en ce que**
   l'augmentation de l'intensité de champ magnétique (B) a lieu chaque fois après des intervalles de temps égaux et/ou

**caractérisé en ce que**
les intensités de champ magnétique pouvant être générées sont comprises entre 0 $\mu$T et 150 $\mu$T et/ou les incréments d'augmentation de l'intensité de champ magnétique sont compris entre 2 $\mu$T et 60 $\mu$T et/ou les intervalles de temps entre les étapes d'augmentation sont compris entre 5 et 8 secondes.

6. Dispositif de magnétothérapie selon l'une des revendications précédentes,
   **caractérisé en ce que**
   l'intensité de champ dans la deuxième période ($T_2$) est de 15 à 40 $\mu$T, de préférence de 20 à 30 $\mu$T, en particulier de 25,12 $\mu$T.

7. Dispositif de magnétothérapie selon l'une des revendications précédentes,

   **caractérisé en ce que**
   la première période ($T_1$) dure de 28 à 58 secondes, de préférence de 38 à 48 secondes, en particulier 43,96 secondes, et/ou
   **caractérisé en ce que**
   un cycle composé de la première ($T_1$) et de la deuxième ($T_2$) période est répété, de préférence 40 à 58 fois, en particulier 49 fois ou 30 à 42 fois, en particulier 37 fois, et/ou
   **caractérisé en ce que**
   la durée totale de l'application est de 50 à 66 minutes, en particulier de 58,09 minutes ou de 30 à 42 minutes, en particulier de 36,75 minutes.

8. Dispositif de magnétothérapie selon l'une des revendications précédentes,
   **caractérisé en ce que**

   le dispositif de génération de champ magnétique comprend au moins une, de préférence au moins deux, en particulier exactement deux, bobine(s) conductrice(s), une première bobine (12) étant de préférence ovale ou elliptique et/ou ayant un diamètre (maximal) de 1,5 à 2,0 mètres, de préférence de 1,70 à 1, 80 mètres, et/ou
   **caractérisé en ce que**
   une deuxième bobine (13) présente de préférence une section transversale ronde et/ou de préférence une section transversale qui est plus petite que la section transversale de la première bobine (12) et/ou
   **caractérisé en ce que**
   une zone centrale d'une ou plusieurs des bobines (12, 13) est disposée dans la région abdominale (plexus solaire) du patient.

9. Dispositif de magnétothérapie selon l'une des revendications précédentes,
   **caractérisé en ce que**

le dispositif de génération de champ magnétique comprend un matelas dans lequel de préférence au moins une bobine (12, 13) est intégrée.

Fig. 1

Fig. 2

15

10

14

Fig. 3

**IN DER BESCHREIBUNG AUFGEFÜHRTE DOKUMENTE**

**In der Beschreibung aufgeführte Patentdokumente**

- DE 102009017229 A1